# FASCICULE DE BREVET EUROPEEN

(11) **EP 1 275 636 B1**
(45) Date de publication et mention de la délivrance du brevet: **02.11.2005**
(21) Numéro de dépôt: 02291692.8
(22) Date de dépôt: 05.07.2002
(51) Int. Cl.: C07C 231/06, C07C 233/09

(54) **Procédé de préparation de méthacrylamide sulfurique**
Verfahren zur Herstellung von Methacrylamidsulfat
Process for preparing methacrylamide sulfate

(30) Priorité: 13.07.2001 FR 0109367
(43) Date de publication de la demande: 15.01.2003
(73) Titulaire: Arkema, 92800 Puteaux (FR)
(72) Inventeur: Croisy Jean-Francois, 37490 L'Hospital (FR); Tosi, Michel, 57660 Lixing les ST Avold (FR)
(74) Mandataire: Hirsch & Associés

(56) Documents cités:
- EP-A- 0 226 724
- EP-A- 0 999 200
- GB-A- 943 536
- GB-A- 1 184 746
- GB-A- 1 186 876
- DATABASE WPI Section Ch, Week 198206 Derwent Publications Ltd., London, GB; Class A41, AN 1982-11531E XP002195031 & SU 825 510 A (SKUGAROVA A V), 30 avril 1981 (1981-04-30)
- DATABASE WPI Section Ch, Week 198243 Derwent Publications Ltd., London, GB; Class E16, AN 1982-92126E XP002195032 & SU 891 646 A (SIVENKOV E A), 23 décembre 1981 (1981-12-23)
- DATABASE WPI Section Ch, Week 197925 Derwent Publications Ltd., London, GB; Class E16, AN 1979-46459B XP002195033 & JP 54 059220 A (MITSUI TOATSU CHEM INC), 12 mai 1979 (1979-05-12)
- DATABASE CA [en ligne] CHEMICAL ABSTRACTS SERVICE, COLUMBUS, OHIO, US; retrieved from STN Database accession no. 83:163604/DN, HCAPLUS XP002195029 & MICHURIN, A.A. ET AL.: ZH. ORG. KHIM., vol. 11, no. 8, 1975, page 1772
- DATABASE CA [en ligne] CHEMICAL ABSTRACTS SERVICE, COLUMBUS, OHIO, US; retrieved from STN Database accession no. 97:110422/DN, HCAPLUS XP002195030 & POLIEVKA, M. ET AL.: PETROCHEMIA, vol. 22, no. 2, 1982, pages 33-38,

## Description

La présente invention concerne un procédé de préparation de méthacrylamide sulfurique à partir de cyanhydrine d'acétone.

Le méthacrylamide sulfurique ou sulfate de méthacrylamide [CH₂=C(CH₃)-CONH₂, H₂SO₄] est un produit intermédiaire qui donne, par réaction avec du méthanol et de l'eau, du méthacrylate de méthyle.

Il est connu de préparer du méthacrylamide sulfurique à partir de cyanhydrine d'acétone et d'acide sulfurique concentré (GB-A 1 184 746 et GB-A 943536).

Cette préparation comprend en général la réaction de la cyanhydrine d'acétone avec de l'acide sulfurique concentré pour donner de l'α-hydroxyisobutyramide et du sulfate d'α-hydroxyisobutyramide. Ces deux composés sont ensuite chauffés pour donner le méthacrylamide sulfurique.

Une telle préparation fait l'objet du brevet français n° 1 529 440 dans lequel est décrit un procédé avec étagement de l'introduction de cyanhydrine d'acétone, comprenant une étape où l'on mélange de la cyanhydrine d'acétone avec de l'acide sulfurique en utilisant un rapport molaire acide sulfurique/cyanhydrine d'acétone au moins égal à 1,5, une étape où l'on soumet le mélange obtenu précédemment à une déshydratation par chauffage, on rajoute ensuite de la cyanhydrine d'acétone au milieu réactionnel de façon à avoir de nouveau un rapport molaire acide sulfurique/cyanhydrine (supérieur à 1) et, finalement, on soumet de nouveau le mélange réactionnel à une déshydratation par chauffage.

La demande de brevet européen n° 226 724 a trait à un procédé de préparation de méthacrylamide à partir de cyanhydrine d'acétone et d'acide sulfurique concentré, dans lequel on introduit dans l'acide sulfurique un mélange liquide à deux phases constitué d'un hydrocarbure saturé linéaire et inerte ayant de 5 à 7 atomes de carbone et de la cyanhydrine d'acétone.

GB-A 1 186 876 décrit un procédé qui utilise l'acide sulfurique contenant un léger excès de trioxyde de soufre dans les étapes.

La demande de brevet européen n° 999 200 se rapporte à un appareillage et un procédé pour la production à haut rendement de méthacrylate de méthyle ou d'acide méthacrylique dans lequel on utilise pour la conversion thermique, un réacteur de craquage du type piston.

Dans la traduction de l'article paru dans le journal russe Zhurnal Prikladnoi Khimii, Vol. 47, No. 6, pp. 1347-1351, June 1974, intitulé « Various reactions occuring in the synthesis of methyl methacrylate from acetone cyanohydrin and oleum », de A. A. Michurin, E. A. Sivenkov, E. N. Zil'berman et T.I. Tret'yakova, il est démontré que la réaction de la cyanhydrine d'acétone avec l'acide sulfurique pour donner le sulfate d'α-hydroxyisobutyramide fournit les meilleurs résultats quand elle est conduite avec un rapport molaire cyanhydride/oléum de 1/2 en présence d'oléum contenant 5 à 10% de trioxyde de soufre. Cependant, pour limiter l'apparition de réactions secondaires lors de l'étape de cuisson ultérieure, il est conseillé d'opérer avec un oléum à 0-3% de trioxyde de soufre.

L'invention a pour but de proposer un procédé de préparation de méthacrylamide sulfurique qui présente un rendement élevé même lorsqu'on utilise un faible excès d'acide sulfurique, c'est-à-dire lorsque le rapport molaire global acide sulfurique/cyanhydrine d'acétone est faible.

Le procédé selon l'invention comprend les étapes suivantes :
a) une étape de mélange, où l'on mélange de la cyanhydrine d'acétone avec de l'acide sulfurique dépourvu de trioxyde de soufre ;
b) une étape de cuisson, où l'on soumet le mélange obtenu précédemment à une déshydratation par chauffage ;
c) une étape de rajout de cyanhydrine d'acétone, où l'on rajoute de la cyanhydrine d'acétone au milieu réactionnel, sans rajouter de l'acide sulfurique, et on mélange ;
d) une autre étape de cuisson, où l'on soumet de nouveau le mélange à une déshydratation par chauffage ;
e) éventuellement, une ou plusieurs étape(s) supplémentaire(s) de rajout de cyanhydrine d'acétone suivie(s) d'une étape supplémentaire de cuisson ;
f) éventuellement, une étape finale de cuisson ;
et il se caractérise en ce qu'on introduit en outre du trioxyde de soufre à l'étape c) et/ou juste après cette étape c) et/ou, le cas échéant, à l'une ou à plusieurs des étapes supplémentaires de rajout e) et/ou juste après cette ou ces étape(s) e).

Un tel procédé a donc l'avantage de réduire le nombre et l'importance des réactions secondaires. En outre, il permet d'utiliser des réactifs de départ qui ne sont pas nécessairement anhydres.

D'autres caractéristiques et avantages de l'invention apparaîtront à la lecture de l'exposé qui suit et qui est donné en référence aux dessins dans lesquels :
- la Figure 1 est un schéma représentant le montage utilisé pour la mise en oeuvre du procédé selon l'invention ;
- la Figure 2 est un graphique représentant l'évolution du rendement en fonction du rapport molaire acide sulfurique/cyanhydrine d'acétone pour le procédé selon l'invention et un procédé de l'art antérieur.

### EXPOSE DETAILLE DE L'INVENTION

L'invention est basée sur la découverte qu'en introduisant du trioxyde de soufre aux points du procédé déjà riches en méthacrylamide, il est possible de convertir chimiquement et quasiment instantanément l'α-hydroxyisobutyramide en sulfate d'α-hydroxyisobutyramide, et que ce dernier se transforme rapidement en méthacrylamide lors de la cuisson ultérieure.

Ainsi, l'introduction de trioxyde de soufre peut-elle être effectuée à l'étape c), c'est-à-dire après l'étape de pré-cuisson, endroit où le rapport molaire RM acide sulfurique/cyanhydrine d'acétone est élevé, ou bien juste après l'étape c), c'est-à-dire en fin d'introduction de la cyanhydrine d'acétone, c'est-à-dire lorsque ledit rapport molaire RM est faible.

De préférence, le trioxyde de soufre est introduit uniquement à l'étape c).

Selon l'invention, il n'est pas nécessaire de travailler avec des réactifs anhydres. Il est possible d'utiliser un acide sulfurique de titre proche de 100% sans viser un titre précis. En outre, une cyanhydrine d'acétone de qualité médiocre peut convenir. On peut par exemple mettre en oeuvre une cyanhydrine d'acétone contenant de 500 à plusieurs milliers de ppm en eau.

Le trioxyde de soufre peut être sous la forme d'oléum, ce qui facilite son introduction.

L'oléum contient généralement plus de 10% et de préférence plus de 15% en poids, de trioxyde de soufre.

Il peut avantageusement correspondre à un oléum industriel, par exemple de titre 104,5 % (équivalent H₂SO₄ - ayant environ 20% en poids de trioxyde de soufre libre) obtenu sur une unité classique de régénération d'acide d'un procédé de fabrication de méthacrylate de méthyle. Ceci est surprenant, car dans l'art antérieur, notamment dans la publication russe précitée, l'usage d'un oléum conduit à de gênantes réactions secondaires.

La quantité d'oléum ajoutée est inférieure à 10% en poids par rapport à la charge totale de cyanhydrine d'acétone et d'acide sulfurique. Elle est de préférence comprise entre 3 et 5% par rapport à cette charge totale.

Ainsi, de tels taux d'oléum injectés restent compatibles avec des rapports molaires RM faibles et ne posent pas de problèmes de viscosité ou de cristallisation, justement parce que l'oléum est injecté après une étape de pré-cuisson, c'est-à-dire à un endroit où il y a déjà une partie du sulfate d'α-hydroxyisobutyramide qui s'est convertie en méthacrylamide.

De plus, aucune exothermie particulière ne se produit au point d'injection de l'oléum, contrairement au cas de l'art antérieur où l'on l'ajoute l'oléum directement à la cyanhydrine d'acétone.

On n'observe aucune cristallisation non plus, qui serait due à la remontée du taux de sulfate d'α-hydroxyisobutyramide, ce qui se produit classiquement quand on injecte de l'oléum avant la pré-cuisson.

Selon l'invention, le trioxyde de soufre est généralement injecté en léger défaut par rapport à l'α-hydroxyisobutyramide à convertir en sulfate d'α-hydroxyisobutyramide. Ce défaut peut correspondre à un rapport molaire trioxyde de soufre/α-hydroxyisobutyramide inférieur à 1 et de l'ordre de 0,8 - 0,9.

Il en résulte qu'il n'y a jamais de trioxyde de soufre libre dans le milieu réactionnel (sortie étape C). Ceci a pour avantage de limiter les réactions secondaires liées au trioxyde de soufre, notamment la formation de diméthylacrylamide, de sultone et de polymères.

Selon l'invention, le rapport molaire RM à l'étape c) est généralement inférieur à 1,7 et de préférence compris entre 1,15 et 1,35.

Pour ce qui est du montage à utiliser pour mettre en oeuvre le procédé selon l'invention, il comprend d'une manière générale, en succession :
- un premier réacteur de mélange Ra,
- un premier réacteur de cuisson Rb,
- un second réacteur de mélange Rc pourvu de moyens d'introduction de cyanhydrine d'acétone,
- un second réacteur de cuisson Rd,
- éventuellement, un ou plusieurs réacteur(s) supplémentaire(s) de mélange suivi(s) d'un réacteur supplémentaire de cuisson, et
- éventuellement, un réacteur final de cuisson Re,
et il se distingue d'un montage classique en ce qu'il comprend, en outre, des moyens d'introduction de trioxyde de soufre dans le réacteur Rc et/ou à la sortie de ce réacteur et/ou, le cas échéant, dans l'un ou plusieurs des réacteurs supplémentaires de mélange et/ou à la sortie de ce ou ces réacteur(s) supplémentaire(s).

Lorsque le montage comprend des réacteurs supplémentaires de mélange, chacun de ces réacteurs est suivi d'un réacteur supplémentaire de cuisson.

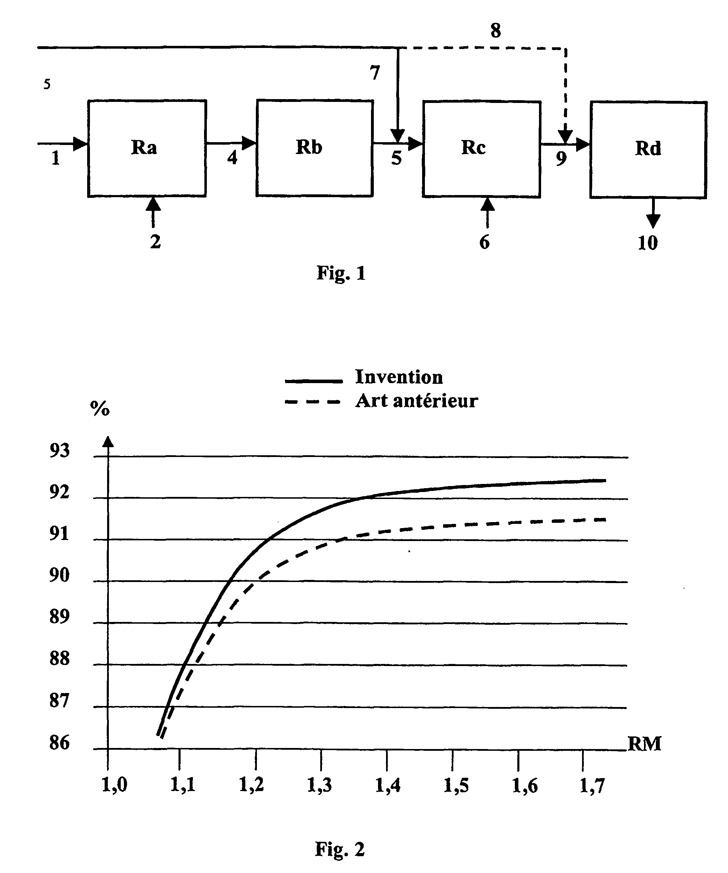

La figure 1 est un schéma illustrant un montage simplifié que l'on peut utiliser pour mettre en oeuvre le procédé selon l'invention.

Le procédé selon l'invention a généralement un mode de fonctionnement en continu que l'on peut décrire comme suit.

L'acide sulfurique est introduit par la ligne 1, la cyanhydrine d'acétone par les lignes 2 et 6. L'étape a) a lieu dans le réacteur Ra.

Le mélange obtenu dans le réacteur Ra est introduit par la ligne 4 dans le réacteur Rb où a lieu l'étape b) de pré-cuisson.

Le produit intermédiaire obtenu à l'étape b) est ensuite introduit par la ligne 5 dans le réacteur Rc où il est mélangé avec de la cyanhydrine d'acétone fraîche arrivant par la ligne 6 et, le cas échéant, avec de l'oléum arrivant par la ligne 7.

Le mélange obtenu dans le réacteur Rc est ensuite introduit par la ligne 9 dans le réacteur Rd.

Le cas échéant, de l'oléum est ajouté par la ligne 8 au mélange sortant du réacteur Rc.

Dans le réacteur Rd a lieu la cuisson de l'étape d).

Le cas échéant, le réacteur Rd peut éventuellement être relié à un réacteur final de cuisson Re.

Après la cuisson, on soutire du réacteur Rd, ou, le cas échéant, du réacteur Re, le sulfate de méthacrylamide que l'on utilise pour fabriquer du méthacrylate de méthyle ou de l'acide méthacrylique.

Les réacteurs de mélange fonctionnement habituellement à une température comprise entre 85 et 105°C et de préférence entre 90 et 95°C.

Les réacteurs de cuisson fonctionnement habituellement à une température comprise entre 120 et 145°C et de préférence entre 120 et 140°C.

Selon un mode de réalisation préféré de l'invention, l'un au moins des réacteurs Rb, Rd et éventuellement Re est un réacteur piston.

### Exemples

Les exemples suivants illustrent l'invention sans toutefois en limiter la portée.

Tous les essais ont été conduits dans un montage tel qu'illustré sur la Figure 1.

Le réacteur Ra était un réacteur agité double enveloppe (d'un volume d'environ 270 ml).

Le réacteur Rb était un réacteur piston chauffé électriquement (d'un volume d'environ 60 ml).

Le réacteur Rc était un réacteur agité double enveloppe (d'un volume d'environ 300 ml).

Le réacteur Rd était un réacteur piston chauffé électriquement (d'un volume d'environ 36 ml).

Un réacteur Re (non représenté) piston et double enveloppe (d'un volume d'environ 240 ml) était relié à la sortie du réacteur Rd.

Les principales caractéristiques des essais étaient les suivantes :
- la réaction se déroulait en continu sur deux étages, plus cuisson finale ;
- la cyanhydrine d'acétone était répartie sur les deux étages (alimentation étagée en cyanhydrine d'acétone) ;
- le rapport molaire RM était ajusté en jouant sur la quantité de cyanhydrine d'acétone introduite dans le réacteur Rc ;
- le débit d'acide sulfurique à 100% était maintenu constant à l'entrée de Ra (environ 570 g/h) et c'est le débit de cyanhydrine d'acétone qui était ajusté suivant le RM final visé en sortie du réacteur piston Re ;
- les titres en acide sulfurique étaient mesurés par potentiométrie (dosage par la soude) ;
- les teneurs en produits formés étaient mesurées par HPLC.

### Essai 1 (comparatif)

Dans cet essai, le rapport RM dans le réacteur Rc était de 1,60 ; la cyanhydrine d'acétone était répartie de la façon suivante : 70% dans le réacteur Ra et 30% dans le réacteur Rc.

Les autres conditions sont regroupées dans le tableau suivant.

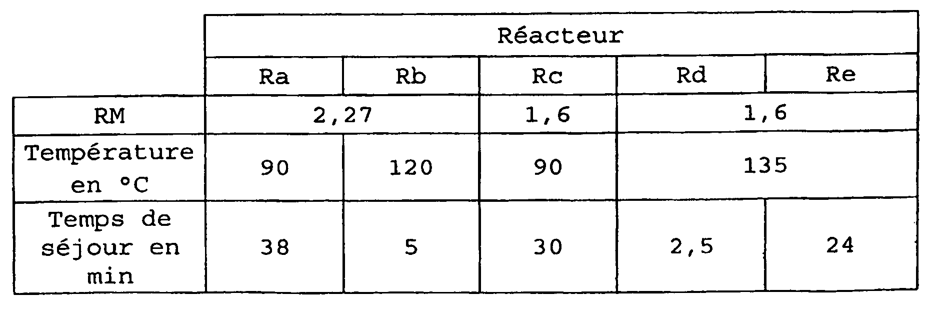

### Essai 2 (invention)

Dans cet essai, le rapport RM dans le réacteur Rc était de 1,50 ; la cyanhydrine d'acétone était répartie de la façon suivante : 70% dans le réacteur Ra et 30% dans le réacteur Rc.

De l'oléum de titre 104,5% était introduit à raison de 45 g/h par la ligne 7 dans le réacteur Rc.

Les autres conditions sont regroupées dans le tableau suivant :

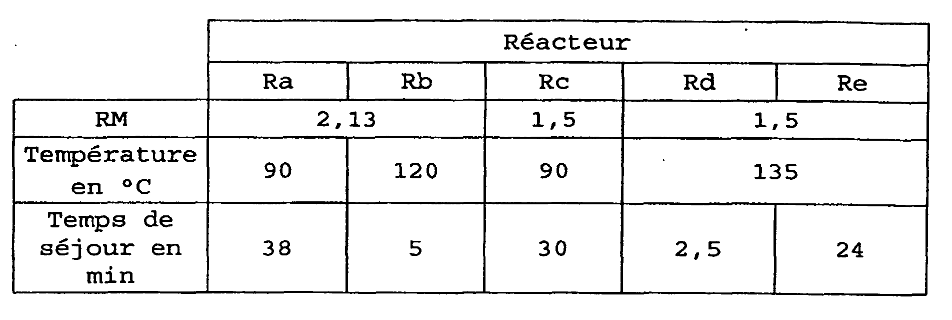

### Essais 3 (comparatif), 3bis (invention) et 3ter (invention)

Dans ces essais, le rapport RM dans le réacteur Rc était de 1,30 ; la cyanhydrine d'acétone était répartie de la façon suivante : 70% dans le réacteur Ra et 30% dans le réacteur Rc.

Dans l'essai 3 bis, de l'oléum de titre 104,5% était en outre introduit à raison de 45 g/h par la ligne 8 à la sortie du réacteur Rc.

Dans l'essai 3 ter, de l'oléum de titre 104,5% était introduit à raison de 45 g/h uniquement par la ligne 7 dans le réacteur Rc.

Les autres conditions sont regroupées dans le tableau suivant :

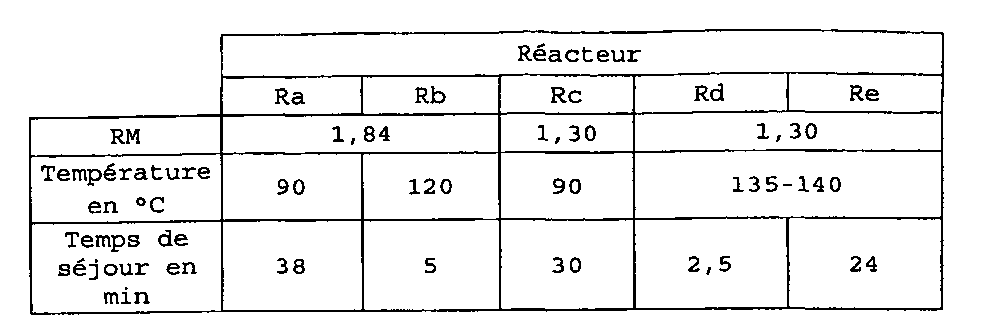

### Essais 4 (comparatif) et 4bis (invention)

Dans ces essais, le rapport RM dans le réacteur Rc était de 1,20 ; la cyanhydrine d'acétone était répartie de la façon suivante : 60% dans le réacteur Ra et 40% dans le réacteur Rc.

Dans l'essai 4bis, de l'oléum de titre 104,5% était en outre introduit à raison de 45 g/h par la ligne 7 dans le réacteur Rc.

Les autres conditions sont regroupées dans le tableau suivant :

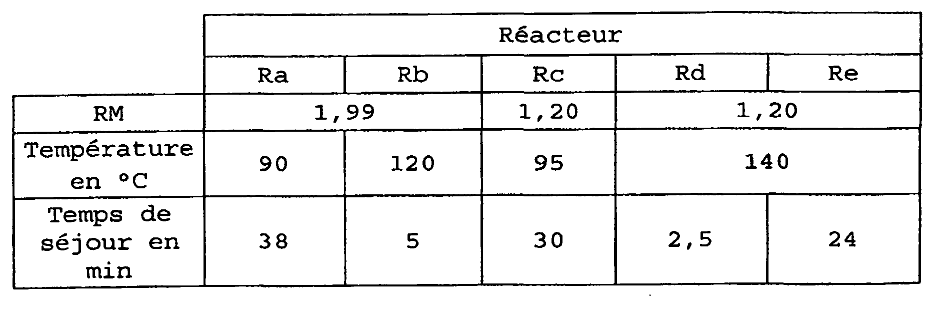

### Essais 5 (comparatif) et 5bis (invention)

Dans ces essais, le rapport RM dans le réacteur Rc était de 1,10 ; la cyanhydrine d'acétone était répartie de la façon suivante : 60% dans le réacteur Ra et 40% dans le réacteur Rc.

Dans l'essai 5bis, de l'oléum de titre 104,5% était en outre introduit à raison de 49 g/h par la ligne 7 dans le réacteur Rc.

Les autres conditions sont regroupées dans le tableau suivant :

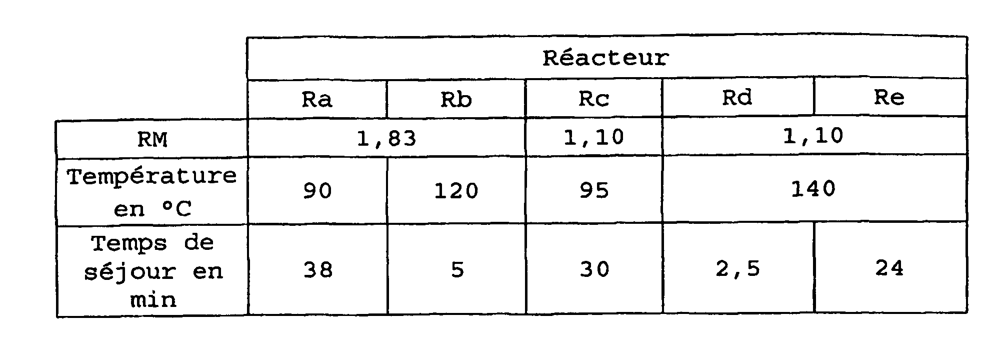

### Résultats

On a calculé les rendements en méthacrylamide obtenus dans tous les essais effectués.

Les résultats sont consignés dans le tableau suivant :

| Essais | RM | Oléum | Rendement en % |
|---|---|---|---|
| 1 | 1,60 | non | 91,50 |
| 2 | 1,50 | oui | 92,24 |
| 3 | 1,30 | non | 91,04 |
| 3bis | 1,30 | oui | 91,45 |
| 3ter | 1,30 | oui | 91,59 |
| 4 | 1,20 | non | 90,05 |
| 4bis | 1,20 | oui | 90,34 |
| 5 | 1,10 | non | 87,31 |
| 5bis | 1,10 | oui | 87,34 |

La courbe de la Figure 2 représente l'évolution des rendements en fonction du rapport molaire RM dans le réacteur Rc.

## Revendications

1. Procédé de préparation de méthacrylamide sulfurique comprenant :
a) une étape de mélange, où l'on mélange de la cyanhydrine d'acétone avec de l'acide sulfurique dépourvu de trioxyde de soufre ;
b) une étape de cuisson, où l'on soumet le mélange obtenu précédemment à une déshydratation par chauffage ;
c) une étape de rajout de cyanhydrine d'acétone, où l'on rajoute de la cyanhydrine d'acétone au milieu réactionnel, sans rajouter de l'acide sulfurique, et on mélange ;
d) une autre étape de cuisson, où l'on soumet de nouveau le mélange à une déshydratation par chauffage ;
e) éventuellement, une ou plusieurs étape(s) supplémentaire(s) de rajout de cyanhydrine d'acétone suivie(s) d'une étape supplémentaire de cuisson ;
f) éventuellement, une étape finale de cuisson ;
**caractérisé en ce qu'**on introduit en outre du trioxyde de soufre à l'étape c) et/ou juste après cette étape c) et/ou, le cas échéant, à l'une ou à plusieurs des étapes supplémentaires de rajout e) et/ou juste après cette ou ces étape(s) e).

2. Procédé selon la revendication 1, dans lequel le trioxyde de soufre est introduit sous forme d'oléum.

3. Procédé selon la revendication 2, dans lequel l'oléum contient plus de 10% et de préférence plus de 15% en poids, de trioxyde de soufre.

4. Procédé selon la revendication 2 ou la revendication 3, dans lequel la quantité d'oléum ajoutée est inférieure à 10% en poids par rapport à la charge totale de cyanhydrine d'acétone et d'acide sulfurique.

5. Procédé selon la revendication 4, dans lequel la quantité d'oléum ajoutée est comprise entre 3 et 5% par rapport à la charge totale de cyanhydrine d'acétone et d'acide sulfurique.

6. Procédé selon l'une des revendications 1 à 5, dans lequel le trioxyde de soufre est introduit uniquement à l'étape c).

7. Procédé selon la revendication 6, dans lequel le rapport molaire acide sulfurique/cyanhydrine d'acétone à l'étape c) est inférieur à 1,7.

8. Procédé selon la revendication 7, dans lequel le rapport molaire acide sulfurique/cyanhydrine d'acétone à l'étape c) est compris entre 1,15 et 1,35.

9. Procédé selon l'une des revendications 1 à 8, dans lequel l'étape b) et/ou l'étape d) est/sont mise(s) en oeuvre dans un réacteur piston.

10. Procédé de fabrication de méthacrylate de méthyle ou de l'acide méthacrylique comprenant la mise en oeuvre du procédé selon l'une des revendications 1 à 9 pour la préparation de méthacrylamide sulfurique et utilisation de ce dernier pour la fabrication dudit méthacrylate de méthyle ou acide méthacrylique.

## Patentansprüche

1. Verfahren zur Herstellung von Methacrylamidsulfat, umfassend:
a) einen Mischschritt, bei dem Acetoncyanhydrin mit Schwefeltrioxid-freier Schwefelsäure gemischt wird;
b) einen Kochschritt, bei dem die vorangehend erhaltene Mischung einer Dehydratisierung durch Erhitzen unterzogen wird;
c) einen Schritt des Hinzufügens von Acetoncyanhydrin, bei dem Acetoncyanhydrin zu dem Reaktionsmedium hinzugefügt wird, ohne Schwefelsäure hinzuzufügen, und gemischt wird;
d) einen weiteren Kochschritt, bei dem die Mischung erneut einer Dehydratisierung durch Erhitzen unterzogen wird;
e) gegebenenfalls einen oder mehrere zusätzlichen (zusätzliche) Schritt(e) der Zugabe von Acetoncyanhydrin, auf einen zusätzlichen Kochschritt folgend;
f) gegebenenfalls einen abschließenden Kochschritt;
**dadurch gekennzeichnet, dass** beim Schritt c) und/oder unmittelbar nach diesem Schritt c) und/oder gegebenenfalls bei dem einen oder den mehreren zusätzlichen Zugabeschritt(en) e) und/oder unmittelbar nach diesem oder diesen Schritt(en) e) ferner Schwefeltrioxid eingeführt wird.

2. Verfahren gemäß Anspruch 1, bei dem der Schwefeltrioxid in Form von Oleum eingeführt wird.

3. Verfahren gemäß Anspruch 2, bei dem das Oleum mehr als 10 Gewichts-%, vorzugsweise mehr als 15 Gewichts-% Schwefeltrioxid enthält.

4. Verfahren gemäß Anspruch 2 oder Anspruch 3, bei dem die Menge des zugegebenen Oleums geringer ist als 10 Gewichts-% im Verhältnis zur Gesamtbeladung von Acetoncyanhydrin und Schwefelsäure.

5. Verfahren gemäß Anspruch 4, bei dem die Menge des zugegebenen Oleums 3 bis 5 % im Verhältnis zur Gesamtbeladung von Acetoncyanhydrin und Schwefelsäure umfasst.

6. Verfahren gemäß einem der Ansprüche 1 bis 5, bei dem das Schwefeltrioxid nur im Schritt c) eingeführt wird.

7. Verfahren gemäß Anspruch 6, bei dem das molare Verhältnis von Schwefelsäure/Acetoncyanhydrin im Schritt c) geringer als 1,7 ist.

8. Verfahren gemäß Anspruch 7, bei dem das molare Verhältnis Schwefelsäure/Acetoncyanhydrin im Schnitt c) 1,15 bis 1,35 beträgt.

9. Verfahren gemäß einem der Ansprüche 1 bis 8, bei dem der Schritt b) und/oder der Schritt d) in einem Druckreaktor ausgeführt wird/werden.

10. Verfahren zur Herstellung von Methylmethacrylat oder von Methacrylsäure, umfassend das Durchführen des Verfahrens gemäß einem der Ansprüche 1 bis 9 zur Herstellung von Methacrylamidsulfat und der Verwendung des Letzteren zur Herstellung des besagten Methylmethacrylats oder der Methacrylsäure.

## Claims

1. Process for preparing sulphuric methacrylamide, comprising:
a) a mixing step, in which acetone cyanohydrin is mixed with sulphuric acid which is free of sulphur trioxide ;
b) a stoving step, in which the mixture obtained above is subjected to a dehydration by heating;
c) a step of adding further acetone cyanohydrin, in which more acetone cyanohydrin is added to the reaction medium, without adding more sulphuric acid, and with mixing;
d) another stoving step, in which the mixture is subjected to a further dehydration by heating;
e) optionally, one or more additional step(s) of further addition of acetone cyanohydrin followed by an additional stoving step;
f) optionally, a final stoving step;
**characterized in that** sulphur trioxide is also introduced in step c) and/or just after this step c) and/or, where appropriate, in one or more of the additional steps of further addition e) and/or just after this or these step(s) e).

2. Process according to Claim 1, in which the sulphur trioxide is introduced in the form of oleum.

3. Process according to Claim 2, in which the oleum contains more than 10% and preferably more than 15% by weight of sulphur trioxide.

4. Process according to Claim 2 or Claim 3, in which the amount of oleum added is less than 10% by weight relative to the total charge of acetone cyanohydrin and of sulphuric acid.

5. Process according to Claim 4, in which the amount of oleum added is between 3% and 5% relative to the total charge of acetone cyanohydrin and of sulphuric acid.

6. Process according to one of Claims 1 to 5, in which the sulphur trioxide is introduced only in step c).

7. Process according to Claim 6, in which the sulphuric acid/acetone cyanohydrin molar ratio in step c) is less than 1.7.

8. Process according to Claim 7, in which the sulphuric acid/acetone cyanohydrin molar ratio in step c) is between 1.15 and 1.35.

9. Process according to one of Claims 1 to 8, in which step b) and/or step d) is/are carried out in a piston reactor.

10. Process for preparing methacrylamide or methacrylic acid comprising the implementation of the process according to one of the Claims 1-9 for the preparation of sulphuric methacrylamide and use of the latter for the preparation of said methacrylamide or methacrylic acid.
